# EUROPEAN PATENT SPECIFICATION

(11) **EP 1 874 311 B1**
(45) Date of publication and mention of the grant of the patent: **05.10.2011**
(21) Application number: 06727482.9
(22) Date of filing: 14.04.2006
(51) Int. Cl.: A61K 31/4422, A61K 31/497, A61K 31/4545, A61P 25/28

(54) **A METHOD FOR PREVENTING, DELAYING OR REVERTING ABNORMAL AMYLOID DEPOSITION**
VERFAHREN ZUR PRÄVENTION, VERZÖGERUNG ODER UMKEHR VON ABNORMER AMYLOID-ABLAGERUNG
METHODE POUR PREVENIR, RETARDER OU INVERSER LA PROGRESSION DE PATHOLOGIES ASSOCIEES A UN DEPOT AMYLOIDE ANORMAL

(30) Priority: 15.04.2005 IT PD20050109; 19.01.2006 IT PD20060014
(43) Date of publication of application: 09.01.2008
(73) Proprietor: Research & Innovation S.p.A., Padua (IT)
(72) Inventor: LEON, ALBERTA, I-35142 Padua (IT); DALLE CARBONARE, MAURIZIO, I-35128 Padua (IT)
(74) Representative: Modiano, Micaela Nadia
(86) International application number: PCT/IB2006/000886
(87) International publication number: WO 2006/109164

(56) References cited:
- EP-A- 0 985 667
- EP-A- 1 123 923
- EP-A- 1 191 021
- WO-A-00/02543
- WO-A-02/078670
- WO-A-03/097067
- WO-A-2004/110354
- WO-A-2005/051389
- DATABASE MEDLINE [Online] US NATIONAL LIBRARY OF MEDICINE (NLM), BETHESDA, MD, US; 1 March 2000 (2000-03-01), SAHUQUILLO J ET AL: "[A controlled, double-blind, randomized pilot clinical trial of nicardipine as compared with a placebo in patients with moderate or severe head injury]" XP002400031 Database accession no. NLM10775962 & REVISTA DE NEUROLOGIA. 2000 MAR 1-15, vol. 30, no. 5, 1 March 2000 (2000-03-01), pages 401-408, ISSN: 0210-0010
- FLORIO T ET AL: "Intracellular calcium rise through L-type calcium channels, as molecular mechanism for prion protein fragment 106-126-induced astroglial proliferation." BIOCHEMICAL AND BIOPHYSICAL RESEARCH COMMUNICATIONS. 12 NOV 1996, vol. 228, no. 2, 12 November 1996 (1996-11-12), pages 397-405, XP002399984 ISSN: 0006-291X
- DATABASE MEDLINE [Online] US NATIONAL LIBRARY OF MEDICINE (NLM), BETHESDA, MD, US; 16 April 2000 (2000-04-16), GONZÁLEZ-GONZÁLEZ J A ET AL: "[A study of the tolerability and effectiveness of nicardipine retard in cognitive deterioration of vascular origin]" XP002400032 Database accession no. NLM10893735 & REVISTA DE NEUROLOGIA. 2000 APR 16-30, vol. 30, no. 8, 16 April 2000 (2000-04-16), pages 719-728, ISSN: 0210-0010
- ABE K ET AL: "Amyloid beta toxicity consists of a Ca(2+)-independent early phase and a Ca(2+)-dependent late phase." JOURNAL OF NEUROCHEMISTRY. NOV 1996, vol. 67, no. 5, November 1996 (1996-11), pages 2074-2078, XP002399985 ISSN: 0022-3042

## Description

### FIELD of the INVENTION

The present invention relates to the pharmacological use of specific compounds pertaining to the class of the dihydropyridines, namely nicardipine, 4 in the prevention or therapy of disease states associated with, or at risk of, cerebral amyloidosis. More specifically, the applicant has surprisingly found that nicardipine is, independently of its known capability to interfere with calcium influx in certain mammalian cells, capable of interfering with formation of the amyloidogenic peptides and/or capable, independently of effects on beta-amyloid (Aβ) production, of augmenting the efflux of the amyloidogenic Aβ peptides from the central nervous system and, hence, is applicable in the prevention or therapy of disease states, such as Alzheimer's disease, cerebral amyloid angiopathies, spongiform encephalopathies, scrapie, typically associated with, or at risk of, cerebral amyloidosis.

### Description of Related Art

Alzheimer's disease (AD) is a chronic neurodegenerative disease, today considered to be one of the most frequent causes of senile dementia. Recent worldwide epidemiological studies Indicate that approximately 18 million subjects are currently affected by dementia, of which 700,000 are in Italy. Current drug therapies ( e.g. donepezil, galantamine, rivastigmine, etc), although of clinical utility in providing symptomological benefits in the early phases of AD, do not arrest the progression of the disease nor are of any significant benefit in advanced stages (Brangman, 2003).

From a neuropathological perspective, one of the most striking neuropathological features of AD is the accumulation of the 4-kDa Aβ peptides, principally Aβ peptides 1-40 and 1-42, as fibrillar deposits in the brain parenchyma (senile plaques) and cerebral vessels. This, together with recent evidences indicating, for example, that
a) accumulation of Aβ peptides in brain and along cerebral vessels (amyloid angiopathy) is invariably associated not only with familial but also sporadic AD, a phenomena now known to occur, at least in part , in disease states typically classified as vascular dementia;
b) the accumulating Aβ peptides are potentially neurotoxic, either directly or indirectly, via actions on the non-neuronal cells found in the brain (e.g. microglial cells), the activation of which is accompanied with production of potentially toxic or inflammatory mediators (e.g. oxygen radicals and pro-inflammatory cytokines etc.);
has to date led to the ever-increasing acknowledgment that alterations in the production and/or disposal of cerebral Aβ peptides profoundly influence the degree and progression of diseases accompanied with cerebral amyloidosis., herein included not only AD. As a consequence, pharmacological strategies aiming to reduce the production and/or accumulation of Aβ peptides in brain have the potential to prevent, retard or revert the progression of the disease states accompanied with, or at risk of, cerebral accumulation of amyloidogenic Aβ peptides, a possibility today shown, at least experimentally, to be of potential clinical value (see review Hardy and Selkoe, 2002).

From a scientific prospective, it is now clear that Aβ peptides in brain are generated from the amyloid precursor protein (APP) by proteolytic processes involving a set of membrane-bound proteases. In fact, two proteolytic enzymes, termed beta- and gamma-secretase, have now been identified and shown to be implicated in the sequential APP cleavage resulting in production of Aβ peptide fragments, herein included the amyloidogenic fragments Aβ 1-40 and 1-42. In addition, a metallo-protease, denominated alpha-secretase, has been identified, the proteolytic action of which counteracts the production of the amyloidogenic fragments because it is directed to an internal site within the Aβ peptide sequence. On the other hand, experiments conducted with genetically-modified cells or transgenic animals carrying mutations associated with rare inherited cases of AD, such as those, for example, found in APP itself, have shown that these mutations cause increased Aβ peptide production, in particular Aβ40/42 production in terms of either absolute levels or with respect to total Aβ (Aβtot), a phenomenon correlated with the rate of amyloid deposition. All this, together with other evidences showing, for example, that Aβ is a normal product of cellular metabolism of APP (Sinha and Lieberburg, 1999), has not only shed light on pathogenetic mechanisms implicated in familial AD but also led to the hypothesis that alterations in APP metabolism occur in disease states associated with cerebral amyloidogenic diseases other than familial AD (e.g. sporadic AD, cerebral amyloid angiopathies etc.). Moreover, studies have, as a consequence and with the availability of experimental models of AD, focused in recent years not only on better comprehension of the physiological pathways implicated the processing of APP in brain but also their regulation, in attempt to provide for novel modes, herein including novel drugs, for intervening in the production/accumulation of the amyloidogenic Aβ peptides in brain (Selkoe, 2002).

Currently, from a pharmacological perspective, ever increasing evidences show that APP metabolism and consequent formation of the amyloidogenic Aβ peptides can be pharmacologically manipulated. For example, agents capable of inhibiting beta- or gamma-secretase activity have been shown to diminish the formation of Aβ peptides, while agents capable of activating protein kinase C, an important phosphorylating enzyme, increase the secretion of soluable APP, an effect due to alpha-secretase stimulation and, as such, preclusion of the formation of the amyloidogenic Aβ peptides (Racchi and Govoni, 2003). Also intriguing is the recent discovery that certain non-steroidal anti-inflammatory drugs (NSAIDS) decrease, more or less selectively, the neuronal production of the amyloidogenic peptide Aβ 1-42 (Weggen et al., 2001; Eriksen et al., 2003). Of particular notworthy is that the Aβ 1-42 lowering effect of the NSAIDS. identified (flurbiprofen, indometacin, ibuprofen) reportedly occurs in a manner independent of their anti-inflammatory action (Eriksen et al., 2003). Rather, recent evidence indicates, surprisingly, that these compounds behave as allosteric modulators of the gamma-secretase enzyme implicated in the formation of the amyloidogenic Aβ peptides (Beher et al., 2004). Other potential therapeutic approaches currently under investigation include substances capable of interfering with the deposition of the amyloidogenic peptides, such as beta-sheet breakers, within the brain itself as well as vaccination procedures or substances aiming to facilitate the efflux of the amyloidogenic peptides from the brain into the blood stream.

WO02/078670 discloses a method and a device for administering a number of drugs, namely neuroprotectants, by cerebrospinal perfusion in pathologies such as stroke, neurodegenerative diseases or trauma, wherein said neurodegenerative diseases comprise, but not are limited to, Alzheimer's disease or multiple sclerosis, whilst the neuroprotectants can be again a long list of products including *inter alia* nicardipine.

WO00/02543 discloses the use of a combination of the AT₁ antagonist valsartan and a calcium channel blocker, that is dihydropyridines or diltiazen-type and verapamil-type non-dihydropyridine agents, for treating a long list of heterogeneous pathologies characterised by vascular disorders, such as for example hypertension, atherosclerosis, renal failure, diabetic retinopathy, cognitive disorders and stroke.

Likewise, WO03/097067 discloses the use of a combination of ACE inhibitor, a calcium channel blocker, that is again dihydropyridines or non-dihydropyridines of diltiazen-type and verapamil-type, and a diuretic for treating the same long list of heterogeneous pathologies based on vascular disorders.

### Summary of the Invention:

the present invention relates to the pharmacological application of a molecule for use to prevent, retard or revert the progression of Alzheimer's disease or other pathologies characterized by abnormal deposition of amylodogenic Aβ peptides. The applicant has surprisingly found that compounds pertaining to the dihydropyridine class of calcium channel blockers are, unlike others, efficacious in decreasing the production/release of the Aβ peptides from brain cells as well as efficacious in augmenting the efflux of amyloidogenic Aβ peptides from brain to blood (see below).
More specifically, the applicant found 1) when employing the neuroglioma cell line stably transfected with human APP carrying mutation associated with Alzheimer's disease, that nicardipine; and niguldipine, at concentrations of 10 and 30 uM, induces robust and significant concentration-dependant inhibition in amount of amyloidogenic Aβ peptide isoforms, assessed via ELISA techniques, accumulating in the culture medium, (Table1), whilst dihydropyridine BayK 8644 shows like some other dihydropyridines nitrendipine and nimodipine (see Facchinetti et al. 2006), no appreciable effect. This, together with the observation that the two stereoisomers of niguldipine, i.e. (+)niguldipine and (-)niguldipine, although known to possess different pharmacological activity as calcium antagonists, display similar efficacy in inhibiting the amount of the amyloidogenic Aβ peptides found in the culture medium, clearly demonstrate that the inhibitory effects observed do not involve their calcium channel bloking properties. Moreover, the applicant found 2) when employing transgenic animals (TG2576 mice) overexpressing human APP bearing the Swedish mutation, that dihydropyridines, such as nicardipine, induce, upon parental administration, augmentation of brain to blood efflux of the cerebral amyloidogenic peptides, an effect reflected by their rapid augmentation in the bloodstream. This effect, together with the observation that these, and not other dihydropyridines known to behave a calcium channel blockers (e.g. nimodipine) also decrease amyloidogenic Aβ peptide formation from neuronal cells in vitro, renders nicardipine ,unexpectedly, of particular utility in disease states associated with cerebral amyloidosis. Indeed, the results obtained sharply contrast with the unsatisfactory conclusions deriving from meta-analysis of clinical studies conducted with dihydropyridines, such as nimodipine, in AD. In fact, it has recently been reported, unlike the molecule here claimed, that the dihydropyridines nimodipine and nitrendipine, although generally considered as prototypes of the calcium channel blockers belonging to the dihydropyridine family, do not significantly decrease the amount of amyloidogenic Aβ peptides found in culture media from a variety of different neuronal cell lines but, rather and in contrast, increase the amount of the beta-amyloid isoform 1-42 (Facchinetti et al., 2006).

In sum, the present invention, identifies a specific molecule pertaining to the dihydropyridine family, such as nicardipine, able unexpectedly to interfere with the formation of amyloidogenic Aβ peptides and/or increase their efflux from the central nervous system and, as such, able to reduce Aβ deposition in brain in disease states associated with, or at risk of, cerebral amyloidosis, such as Alzheimer's disease, cerebral amyloid angiopathies, spongiform encephalopathies and scrapie. Moreover, the present invention refers to the use of the above mentioned compound. The present invention also relates to uses of compositions of the molecule, herein claimed, in association with other pharmacologically active substances capable of affecting amyloid burden in brain, such as anti-oxidants, anti-inflammatory agents, protease inhibitors, acetylcholine esterase inhibitors, etc.

### DESCRIPTION OF THE INVENTION

The aims and the advantages deriving from the medical use of the dihydropyridines nicardipine, object of the present invention, in pathological conditions characterized by cerebral amyloidosis, will be better explained in the following detailed description of the invention. Applicant, in fact, has for the first time found that treatment with the compound, object of the present invention, significantly reduces, in contrast to other dihydropyridines clinically in use (e.g. nimodipine, nitrendipine) as calcium channel blockers, the amount of amyloidogenic Aβ peptides accumulating in culture media of human transfected cell lines expressing an isoform of the APP695 gene associated with Alzheimer disease. This effect does not, as exemplified by the capability of the two stereoisomers of niguldipine, i.e. (+)niguldipine and (-)niguldipine to exert, unlike their reported dissimilar pharmacological efficacy as calcium channel antagonists, similar efficacy in inhibiting the amount of the amyloidogenic Aβ peptides found in the culture medium, involve action of these compounds on voltage-gated calcium channels and as such are of absolute novelty.

In addition, the Applicant has found that the treatment of transgenic animals (TG2576 mice), overexpressing human APP bearing the Swedish mutation, with the compound object of the present invention, induces a significant increase in the concentration of amyloidogenic peptides in the blood stream of the treated animals, an effect evident already at 3 hours following their administration. This, given that proteolytic processing of APP occurs mainly, if not entirely, within the brain, reflects increased efflux of these peptides from brain tissue where they are known to accumulate in different pathological conditions.

### Example 1

Human neuroglioma (H4) cells, stably transfected with the gene codifying for the human protein APP695 having mutations related to Alzheimer disease, have been used. The expression of APP695 protein is under the control of a constitutively active promoter.

Cells were grown in OPTI-MEM containing 10% FBS (foetal bovine serum), hygromycin (0.2 mg/ml) and blasticidine (0.002 mg/ml).

The Aβ peptide in cells is produced by the sequential processing of beta- and gamma-secretases proteases and is then secreted in the culture medium.

Peptide concentration in the extracellular medium was measured using a specific ELISA methodology. As shown in Table 1, treatment of cells with 10 - 30 µM of different dihydropyridines considered was found to be accompanied by a significant reduction in the amount of the Aβ peptides found in the culture medium. In these conditions, cell survival, as assessed biochemically employing the traditional MTT assay, was not affected.

Given that dihydropyrides typical occur in racemic mixtures, with only one stereoisomer possessing calcium channel blocking activity, the applicant also investigated the effect of the single enantiomer. As exemplified in Table 1, both (+)niguldipine and (-) niguldipine were found efficacious in reducing the extracellular amount of Aβ peptides (57% and 53% inhibition at 30 µM respectively). Of noteworthy is that, the enatiomer (-)niguldipine is, in contrast to (+)niguldipine, reportedly not active as calcium channel antagonist. Moreover, the dihydropyridine BayK 8644, unlike nicardipine, and niguldipine, did not affect beta-amyloid content in culture media from H4/APP cells (Tab. 1), indicating that some, but not all dihydropyridines, are endowed with the Aβ lowering effect.

### Example 2.

The selected dihydropyridine, nicardipine (see table 2) as well as nimodipine were administered, in TG2576 animals, at dose of 10 mg/Kg i.p. Animals were bleed before treatment for assessment of basal circulating Aβ peptides as well as 3 hours post-treatment. Determination of blood amyloidogenic Aβ peptide concentration was performed using ELISA technique. Results, reported in table 2, show that the administration of nicardipine causes significant elevation in the amount of circulating amyloidogenic Aβ peptides, a result indicative of increased efflux from brain tissue of these peptides.

Surprisingly, nicardipine causes, in sharp contrast to its capability to decrease the formation of amyloidogenic Aβ peptides in vitro, elevations in circulating total amyloidogenic Aβ peptides. In contrast nimodipine, previously reported to selectively elevate Aβ 1-42 both in vitro and in vivo (see also Facchinetti et al 2006), does not affect total blood levels of the amyloidogenic Aβ peptides. As circulating amyloidogenic Aβ peptides are generally considered to be of brain origin, this indicate that some, but not other, dihydropyridines augment the efflux of these peptides from brain to blood.

In the present invention the Applicant claims the dihydropyridine nicardipine, for use in the treatment of patients affected with pathologies such as Alzheimer disease, cerebral amyloid angiopathies or other pathologies characterised by an abnormal deposition of amyloidogenic Aβ peptides.

The molecule, object of the present invention, can be employed, alone or in association with other selected therapeutic agents, for preparation of pharmaceutical compositions useful for specific therapeutic purposes. The selected therapeutic agents to be used in combination with the molecules object of the present invention, can be chosen among: anti-oxidants, protease inhibitors, acetylcholine esterase inhibitors, anti-convulsivants, neuroleptics, atypical neuroleptics, anti-depressants, dopamine-agonists, gaba-agonists, drugs for memory improvement, anti-inflammatory drugs, pain-killers (eg. opiods, salycilates, pyrazolics, indolics, anthranilics, aryl-propyonics, aryl-acetics, oxicams, pyranocarboxilics, glucocorticoids, cox2 inhibitors, and acetaminophen).

The administration routes that can be used for the preventive or therapeutic treatment can be oral, parenteral, topical, transdermical, rectal, sublingual or nasal administration. The compound nicardipine, object of the present invention, can be administered in pharmaceutical compositions in combination with excipients, dispersants and diluents, known or new, compatible with their pharmaceutical use, with the aim to obtain a better delivery of the active substances to the site of action and a fast or sustained or slow release. Dosage are dependant on the severity of the pathology and on the route of administration, as well as on the physical status of the patients (age, body weight, general health conditions). As an example, but not limiting one, dosages may be from 0.1 to 50 mg/kg of body weight as daily repeated administrations for a period between 2 to 16 weeks. For oral administration, dispersible powders, tablets, hard or soft gelatine capsules, suspensions, emulsions or solutions can be used. For parenteral administration, buffered water solutions or oily solutions or suspensions, or ready-to-use dry lyophilised dispersible powders, can be employed. For topical, transdermal, rectal, sublingual or nasal administration, said compositions may be in the form of plaster, suppository, ovules, aerosol, spry, emulsions, suspensions or solutions.

### BIBLIOGRAPHY

1. Brangman SA. 2003 Long-term cholinesterase inhibitor therapy for Alzheimer's disease: implications for long-term care. Am J Alzheimers Dis Other Demen. 18(2):79-84
2. Beher D, Elle C, Underwood J, Davis JB, Ward R, Karran E, Masters CL, Beyreuther K, Multhaup G. 1999 Proteolytic fragments of Alzheimer's disease-associated presenilin 1 are present in synaptic organelles and growth cone membranes of rat brain. J Neurochem. 72(4):1564-73
3. Eriksen JL, Sagi SA, Smith TE, Weggen S, Das P, McLendon DC, Ozols W, Jessing KW, Zavitz KH, Koo EH, Golde TE. 2003. NSAIDs and enantiomers of flurbiprofen target gamma-secretase and lower Abeta 42 in vivo. J Clin Invest.; 112(3):440-9.
4. Esler WP, Wolfe MS. 2001. A portrait of Alzheimer secretases--new features and familiar faces. Science. 293(5534):1449-54.
5. Facchinetti F, Fasolato C., Del Giudice E, Burgo A., Furegato S., Fusco M., Basso E., Seraglia R., D' Arrigo A. and Leon A. 2006. Nimodipine selectively stimulates beta-amyloid 1-42 secretion by a mechanism independent of calcium influx blockage. Neurobiol. Aging 27(2):218-27
6. Hardy J, Selkoe DJ. (2002) The amyloid hypothesis of Alzheimer's disease: progress and problems on the road to therapeutics. Science. 297(5580):353-6.
7. Murphy MP, Hickman LJ, Eckman CB, Uljon SN, Wang R, Golde TE. 1999. gamma-Secretase, evidence for multiple proteolytic activities and influence of membrane positioning of substrate on generation of amyloid beta peptides of varying length. J Biol Chem. 274(17):11914-23
8. Racchi M, Govoni S. 2003 The pharmacology of amyloid precursor protein processing. Exp Gerontol.38(1-2):145-57
9. Selkoe DJ. 2002. Deciphering the genesis and fate of amyloid beta-protein yields novel therapies for Alzheimer disease. J Clin Invest. 110(10):1375-81
10. Sinha S, Lieberburg 1. 1999 Cellular mechanisms of beta-amyloid production and secretion. Proc Natl Acad Sci U S A. 96(20):11049-53
11. Weggen S, Eriksen JL, Das P, Sagi SA, Wang R, Pietrzik CU, Findlay KA, Smith TE, Murphy MP, Bulter T, Kang DE, Marquez-Sterling N, Golde TE, Koo EH. 2001 A subset of NSAIDs lower amyloidogenic Abeta42 independently of cyclooxygenase activity. Nature 414(6860):212-6

### TABLES

**Table 1 Effect of different dihydropyridines on the secretion of total beta-amyloid. Confluent H4/APP cells where incubated for 2 h with the vehicle (0.1 % DMSO) or with nicardipine at different concentrations (3µM, 10µM, 30µM), beta-amyloid concentration in culture medium is expressed as % control**

| Treatment | Dose | % |
|---|---|---|
| Vehicle | | 100 |
| Nicardipine | 10µM | 62* |
| | 30µM | 58* |
| (+)Niguldipine | 10µM | 71* |
| | 30µM | 43* |
| (-)Niguldipine | 10µM | 68* |
| | 30µM | 47* |
| BayK 8644 | 10µM | 98 |
| | 30µM | 96 |

| | | |
|---|---|---|
| *p<0.01 treated vs vehicle. Dunnett test after ANOVA. | | |

**Table 2 Levels of the amyloidogenic peptides in plasma of TG2576 mice before and 3 hours following administration of the exemplified dihydropyridines**

| | Total Aβ (pM) | Total Aβ (%) | Aβ1-42 (pM) | Aβ1-42 (%) |
|---|---|---|---|---|
| Before treatment (n=7) | 869,1±134,1 | 100 | 38.1±4,4 | 100 |
| Nimodipine 10 mg/kg (n=7) | 759,1±27,0 | 87 | 59,5*±5,6 | 156 |
| Before treatment (n=7) | 1000,44±224,9 | 100 | 54,85±9,5 | 100 |
| Nicardipine 10 mg/kg (n=7) | 1702,20*±20 4,9 | 170 | 97,11*±16,0 | 176 |

| | | | | |
|---|---|---|---|---|
| * p<0.01 treatment vs control (T-test) | | | | |

## Claims

1. Use of nicardipine in the preparation of pharmaceutical compositions for the preventive or therapeutic treatment of conditions or disorders associated with cerebral amyloidogenic diseases selected from the group consisting of Alzheimer's disease, cerebral amyloid angiopathies, spongiform encephalopathies and scrapie.

2. Use according to claim 1, wherein said compositions are suitable for oral, parenteral, topical, transdermical, rectal, sublingual or nasal administration.

3. Use according to claim 2, wherein for the oral administration said compositions may be dispersible powders, tablets, hard or soft gelatine capsules, suspensions, emulsions or solutions.

4. Use according to claim 2, wherein the parenteral administration can be intramuscular, sub-cutaneous, intra-venous, peridural and said compositions may be in buffered water or oily solutions or suspensions, or as dry, lyophilised, ready to use dispersible powders.

5. Use according to claim 2, wherein for topical, transdermical, rectal, sublingual or nasal administration said compositions may be in the form of plaster, suppository, ovules, aerosol, spry, emulsions, suspensions or solutions.

## Patentansprüche

1. Verwendung von Nicardipin zur Herstellung pharmazeutischer Zusammensetzungen zur die präventive oder therapeutische Behandlung von Krankheitszuständen oder Erkrankungen, die mit zerebraler Amyloidose assoziiert sind, ausgewählt aus der Gruppe, bestehend aus Alzheimer-Erkrankung, zerebraler Amyloidangiopathie, spongiformen Encephalopathien und Scrapie.

2. Verwendung gemäß Anspruch 1, wobei genannte Zusammensetzungen für orale, parenterale, topische, transdermale, rektale, sublinguale oder nasale Verabreichung geeignet sind.

3. Verwendung gemäß Anspruch 2, wobei die genannten Zusammensetzungen zur oralen Verabreichung in Form von dispergierten Pulvern, Tabletten, Hart- oder Weichgelatinekapseln, Suspensionen, Emulsionen oder Lösungen vorliegen können.

4. Verwendung gemäß Anspruch 2, wobei die parenterale Verabreichung intxaxzxuslculär, subkutan, intravenös und peridural erfolgen kann und die genannten Zusammensetzungen in gepuffertem Wasser oder öligen Lösungen oder Suspensionen oder in Form von trockenen oder lyophilisierten gebrauchsfertigen dispergierten Pulvern vorliegen können.

5. Verwendung gemäß Anspruch 2, wobei die genannten Zusammensetzungen für die topische, transdermale, rektale, sublinguale oder nasale Verabreichung in der Form von Pflaster, Suppositorien (Zäpfchen), Ovula (Vaginalzäpfchen), Aerosol, Spray, Emulsionen, Suspensionen oder Lösungen vorliegen können.

## Revendications

1. Utilisation de nicardipine dans la préparation de compositions pharmaceutiques destinées au traitement préventif ou thérapeutique d'états ou de troubles associés aux maladies amyloïdogéniques cérébrales choisies dans le groupe constitué par la maladie d'Alzheimer, les angiopathies amyloïdes cérébrales, les encéphalopathies spongiformes et la tremblante.

2. Utilisation selon la revendication 1, dans laquelle lesdites compositions sont appropriées pour une administration par voie orale, parentérale, topique, transdermique, rectale, sublinguale ou nasale.

3. Utilisation selon la revendication 2, dans laquelle, pour l'administration par voie orale, lesdites compositions peuvent être des poudres dispersibles, des comprimés, des capsules de gélatine dure ou molle, des suspensions, des émulsions ou des solutions.

4. Utilisation selon la revendication 2, dans laquelle l'administration parentérale peut être intramusculaire, sous-cutané, intraveineuse, péridurale et lesdites compositions peuvent être dans des solutions ou suspensions aqueuses ou huileuses tamponnées, ou sous la forme de poudres dispersibles sèches, lyophilisées et prêtes à l'emploi.

5. Utilisation selon la revendication 2, dans laquelle, pour une administration par voie topique, transdermique, rectale, sublinguale ou nasale, lesdites compositions peuvent être sous la forme d'un emplâtre, d'un suppositoire, d'ovules, d'un aérosol, d'une pulvérisation, d'émulsions, de suspensions ou de solutions.
